# FASCICULE DE BREVET EUROPEEN

(11) **EP 2 878 332 B1**
(45) Date de publication et mention de la délivrance du brevet: **20.04.2016**
(21) Numéro de dépôt: 14191421.8
(22) Date de dépôt: 03.11.2014
(51) Int. Cl.: A61N 1/05

(54) **Microsonde de détection/stimulation implantable dans un vaisseau du réseau veineux, artériel ou lymphatique**
Detektions-/Stimulationsmikrosonde zur Implantation in ein Gefäß des Venen-, Arterien- oder Lymphsystems
Detection/stimulation microprobe implantable in a vessel of the venous, lymphatic or arterial network

(30) Priorité: 27.11.2013 FR 1361708
(43) Date de publication de la demande: 03.06.2015
(73) Titulaire: Sorin CRM SAS, 92143 Clamart Cedex (FR)
(72) Inventeur: Regnier, Willy, 91160 Longjumeau (FR); D'Hiver, Philippe, 92320 Chatillon (FR)
(74) Mandataire: Dupuis-Latour, Dominique

(56) Documents cités:
- EP-A1- 2 455 131
- EP-A1- 2 559 453
- EP-A1- 2 581 107
- WO-A2-02/18006

## Description

L'invention concerne de façon générale les "dispositifs médicaux implantables actifs" tels que définis par la directive 90/385/CEE du 20 juin 1990 du Conseil des communautés européennes.

Cette définition inclut en particulier les implants cardiaques chargés de surveiller l'activité cardiaque et de générer des impulsions de stimulation, de défibrillation et/ou de resynchronisation en cas de trouble du rythme détecté par l'appareil. Elle inclut aussi les appareils neurologiques, les implants cochléaires, les pompes de diffusion de substances médicales, les capteurs biologiques implantés, etc.

Ces dispositifs comportent un boitier généralement désigné "générateur", raccordé électriquement et mécaniquement à une ou plusieurs "sondes" intracorporelles munie(s) d'électrodes destinées à venir en contact avec les tissus sur lesquels on souhaite appliquer des impulsions de stimulation et/ou recueillir un signal électrique : myocarde, nerf, muscle...

La présente invention concerne plus précisément une microsonde de détection/stimulation destinée à être implantée dans des réseaux veineux, artériels ou lymphatiques.

On décrira plus précisément dans la présente demande une application à la stimulation d'une cavité cardiaque par une sonde implantée dans le réseau coronaire, mais cette application n'est aucunement limitative, et la microsonde de l'invention peut être utilisée dans un grand nombre d'autres configurations et applications permises par son très faible diamètre. Dans cet exemple des sondes coronaires destinées à stimuler une cavité gauche du coeur, oreillette ou ventricule, la sonde est introduite non pas dans la cavité à stimuler mais dans le réseau coronarien, et elle est pourvue d'une électrode destinée à venir en contact avec la paroi de l'épicarde au niveau du ventricule gauche ou de l'oreillette gauche, selon le cas. Ces sondes stimulent ainsi le muscle cardiaque via une ou plusieurs électrodes ponctuelles dont la position est fonction de la trajectoire prédéfinie de la veine canulée.

La sonde n'étant pas placée à l'intérieur de la cavité mais contre une paroi, on comprendra l'importance d'une orientation correcte du champ électrique généré par l'électrode, de manière à garantir l'orientation des champs électriques vers le muscle cardiaque par l'intermédiaire de la paroi veineuse, afin de réduire le seuil de stimulation et, par voie de conséquence, l'énergie nécessaire à la stimulation.

Avec des sondes conventionnelles, par exemple le modèle *Situs LV* commercialisé par Sorin CRM, Clamart, France et qui est décrit notamment dans le EP 0 993 840 A1 (ELA Médical), la sonde stimule la cavité par l'intermédiaire d'une électrode en forme de bague annulaire bloquée dans la veine et en contact sur l'ensemble de la périphérie de celle-ci dans la région du site de stimulation. Le champ électrique est alors diffusé dans toutes les directions sur 360°, ce qui correspond à un rayonnement de type dit "annulaire". Une partie du rayonnement sera nécessairement orientée vers le muscle cardiaque du fait du contact permanent avec la veine, ce qui garantit la délivrance de l'énergie de stimulation de la cavité à l'endroit où a été implantée l'électrode. En revanche, une partie non négligeable du champ électrique n'est pas utilisée de façon optimale, car il est dirigé à l'opposé du muscle cardiaque, ce qui correspond à une dépense d'énergie inutile pour la stimulation. En effet, la majeure partie du champ électrique est diffusée dans le sang, dont la conductivité électrique est supérieure à celle du tissu musculaire, conduisant ainsi à de mauvaises performances électriques pour la stimulation cardiaque.

Le WO 02/18006 A2 décrit une sonde d'un type similaire, où l'extrémité distale comporte une préforme hélicoïdale inscrite dont le diamètre est compris entre 2,5 et 20 mm, avec des électrodes régulièrement disposées à 120° sur cette préforme. La configuration de cette préforme permet notamment d'assurer un contact effectif et stable à l'interface entre la sonde et la paroi de vaisseaux de grand diamètre, la préforme hélicoïdale venant mécaniquement, par élasticité, plaquer la sonde contre la paroi intérieure du vaisseau.

Une tendance récente en matière de sonde de stimulation implantable dans un réseau veineux, artériel ou lymphatique est la réduction du diamètre, typiquement jusqu'à un diamètre inférieur à 2 French (0,66 mm), voire jusqu'à 0,5 French (0,17 mm), bien plus faible que les sondes conventionnelles telles que le modèle *Situs LV* décrit plus haut dont le diamètre de la partie active est de l'ordre de 4 à 7 French (1,33 mm à 2,33 mm) ou les sondes telles que celles décrites par le WO 02/18006 A2 précité.

La taille du corps de sonde est en effet un facteur directement lié aux capacités de guidage contrôlé de la sonde, par exemple dans le réseau veineux coronarien, ce qui permet de sélectionner des sites de stimulation particuliers situés dans certaines veines collatérales : le très faible diamètre extérieur de l'extrémité distale active de la sonde permet ainsi de canuler des veines très étroites du réseau coronarien, non exploitées à ce jour du fait de la taille excessive des sondes coronaires conventionnelles.

De telles sondes, que l'on peut qualifier de "microsondes" sont par exemple décrites dans les EP 2 455 131 A1, 2 559 453 A1 et 2 581 107 A1, tous trois au nom de Sorin CRM SAS. La partie active de ces microsondes est constituée par un microcâble présentant un diamètre de l'ordre de 0,5 à 2 French (0,17 à 0,66 mm) comportant une pluralité de parties dénudées formant une succession d'électrodes individuelles, constituant ensemble un réseau d'électrodes reliées en série permettant de multiplier les points de stimulation dans une zone profonde du réseau coronarien.

Comme cela est notamment décrit dans le EP 2 559 453 A1 précité, le très faible diamètre du microcâble permet même d'introduire celui-ci dans une première veine (veine "aller") puis par une anastomose vers une deuxième veine (veine "retour") en remontant dans celle-ci. On a en effet constaté la présence très fréquente d'anastomoses distales dans le réseau veineux coronarien, c'est-à-dire qu'il existe à l'extrémité de certaines veines un passage vers une autre veine, avec de ce fait possibilité de communication entre deux veines distinctes au niveau de l'anastomose, via leurs extrémités distales respectives. Il devient ainsi possible, avec une unique sonde, de stimuler concomitamment deux zones relativement distantes, car situées dans deux veines distinctes. Le double effet d'éloignement de ces deux zones et de multiplication des points de stimulation dans chacune des zones procure un effet particulièrement bénéfique pour la resynchronisation du fonctionnement du coeur.

Un autre avantage du faible diamètre de la partie active de la sonde est qu'il évite l'obstruction d'une partie du flux sanguin dans la veine, qui entrainerait une déficience de l'irrigation du réseau veineux situé en aval de l'extrémité de la sonde.

La réduction du diamètre de la sonde n'est toutefois pas dépourvue d'inconvénients.

En effet, lorsque le diamètre de la sonde est notablement inférieur à celui de la veine, il est difficile de garantir le contact permanent des électrodes. La partie dénudée du microcâble, qui forme une électrode, peut ainsi se trouver dans une position intermédiaire, "flottante", au milieu de la veine, les points de contact entre le microcâble et la paroi de la veine se faisant sur des régions électriquement isolées.

Ce phénomène est particulièrement marqué dans le cas des microcâbles passés par une anastomose : en effet, si les veines sont de faible diamètre dans la région de l'anastomose, typiquement moins de 1 French (0,33 mm), au-delà de l'anastomose elles peuvent rejoindre le sinus coronaire après avoir contourné le ventricule gauche, et dans ce cas leur diamètre augmente. Le microcâble très fin, qui a permis de passer l'anastomose, risque alors d'évoluer dans une région de diamètre relativement large, avec de ce fait une difficulté à établir un contact stable entre les électrodes et la paroi de la veine dans cette région.

Cet inconvénient (absence de contact garanti) est la contrepartie de l'avantage indiqué plus haut d'éviter l'obstruction du flux sanguin dans la veine. *A contrario,* les sondes de plus grand diamètre comprenant une bague de stimulation annulaire bloquée sur toute la périphérie du vaisseau garantissent le contact avec les tissus cibles, mais entraînent nécessairement une oblitération de ce vaisseau qui peut avoir des effets délétères.

Par ailleurs, du point de vue électrique, il importe de garantir, d'une part, l'efficacité de la stimulation malgré un vaisseau de plus grand diamètre que le microcâble de stimulation et, d'autre part, une consommation électrique optimisée même avec une configuration de seulement trois électrodes orientés à 120° applicable dans les anastomoses.

Plus précisément, l'un des buts de l'invention est de proposer une structure de microsonde qui procure en toute circonstance une stimulation efficace malgré un diamètre du microcâble très inférieur à la dimension du vaisseau, et qui :
- garantisse en toute circonstance le contact entre les tissus du vaisseau et les électrodes de stimulation de la microsonde ;
- permette d'orienter le champ électrique en direction du tissu cible à stimuler (le muscle cardiaque) de manière à optimiser les performances électriques en évitant de dissiper une partie non négligeable de l'énergie de stimulation inutilement ; et
- assure une répartition des électrodes le long de la microsonde dans des régions de stimulation bien localisées, en maximisant l'impédance globale de la sonde de manière à réduire le seuil de stimulation et donc l'énergie consommée.

Par ailleurs, ces avantages électriques doivent s'accompagner, sur le plan mécanique, des caractéristiques suivantes :
- la zone stimulante du microcâble doit rester dans un diamètre extérieur typique de l'ordre de 1,5 French (0,5 mm) pour garantir le passage dans les anastomoses, ainsi que dans le microcathéter qui sera utilisé lors de l'implantation, avec un profil "isodiamétrique", c'est-à-dire un diamètre régulier sur toute la longueur de la partie active de la microsonde ;
- la partie active distale doit être suffisamment souple pour ne pas blesser la veine, et doit assurer un contact permanent avec la paroi intérieure du vaisseau afin de réduire les risques de déplacement ou de perte de stimulation ; et
- l'effort de contact entre l'électrode et la paroi intérieure du vaisseau doit être le plus faible possible, idéalement un simple contact affleurant, afin de réduire les risques d'inflammation et de thrombose au point de contact entre le microcâble et le vaisseau.

L'invention propose à cet effet une microsonde de détection/stimulation du type général divulgué par le EP 2 581 107 A1 précité, c'est à dire comportant une partie active distale constituée par un microcâble de diamètre au plus égal à 2 French (0,66 mm) comprenant un coeur électriquement conducteur revêtu d'une couche d'isolement. Ce microcâble comprend au moins une zone de stimulation où la couche d'isolement présente une pluralité de zones dénudées formant des électrodes de stimulation respectives, et présente dans la zone de stimulation une préforme tridimensionnelle configurée de manière que le microcâble épouse la paroi du vaisseau cible dans la zone de stimulation. La préforme tridimensionnelle est inscrite dans un volume enveloppe cylindrique dont le diamètre est choisi pour être, à l'état libre du microcâble, supérieur au diamètre du vaisseau cible.

De façon caractéristique de l'invention, le microcâble comporte dans la région de la préforme tridimensionnelle au moins trois des zones dénudées, ces zones étant disposées en des emplacements respectifs du microcâble situés sur le volume enveloppe cylindrique, ces emplacements étant en outre régulièrement répartis sur la circonférence du volume enveloppe cylindrique considéré en projection axiale. De plus, la surface dépourvue de revêtement isolant de chaque zone dénudée est inférieure à 0,3 mm².

Cette configuration spécifique, propre à l'invention, permet de cumuler sur le plan électrique les avantages suivants :
- les surfaces des électrodes étant réduites, l'impédance Z augmente et la puissance diminue pour une tension donnée V de stimulation (optimisation du paramètre V²/Z) ;
- la répartition uniforme des trois électrodes sur 360° garantit que l'une au moins d'entre elles est toujours orientée au mieux vers le tissu excitable, réduisant ainsi la distance à ce tissu ce qui réduit également la puissance nécessaire pour stimuler ;
- cette électrode la mieux orientée est en contact avec la paroi veineuse avec une pression de contact minimale, ce qui minimise l'inflammation et donc réduit l'élévation progressive du seuil de stimulation, avec un effet favorable à long terme sur la consommation ;
- le nombre de trois électrodes par zone ainsi que leur éloignement limite les pertes d'efficacité liées à la réduction du courant émis du fait de leurs mutuelles influences.

Selon diverses caractéristiques subsidiaires avantageuses :
- la surface dépourvue de revêtement isolant de chaque zone dénudée est inférieure à 0,1 mm² ;
- le microcâble comporte trois zones dénudées disposées en des emplacements respectifs répartis à 120° sur la circonférence du volume enveloppe cylindrique considéré en projection axiale ;
- les zones dénudées ne s'étendent que sur un secteur angulaire du microcâble considéré en section droite, et le secteur angulaire est tourné vers l'extérieur du volume enveloppe de la préforme ;
- la préforme tridimensionnelle est une préforme hélicoïdale ;
- les au moins trois zones dénudées sont disposées en des emplacements respectifs du microcâble régulièrement répartis en direction longitudinale sur le volume enveloppe cylindrique ; et/ou
- la microsonde comprend deux zones de stimulation distinctes séparées par une zone intercalaire d'élongation et de rétention, conformée de manière à rendre la microsonde élastiquement déformable dans le sens longitudinal sous l'effet des sollicitations de traction/compression axiale exercée sur la microsonde dans sa région proximale par rapport à la zone d'élongation.

On va maintenant décrire un exemple de mise en oeuvre de l'invention, en référence aux dessins annexés où les mêmes références désignent d'une figure à l'autre des éléments identiques ou fonctionnellement semblables.
La Figure 1 illustre de façon générale le myocarde, avec les principales veines du réseau coronaire dans lequel a été introduite une sonde selon l'invention, destinée à la stimulation du ventricule gauche.
La Figure 2 illustre la partie d'extrémité du microcâble selon l'invention permettant la stimulation multizone.
La Figure 3 est une vue de bout, dans une direction axiale, de la microsonde de l'invention introduite dans un vaisseau du réseau coronaire, montrant la manière dont le contact est assuré au site de stimulation et dont le champ électrique peut être diffusé spécifiquement en direction du muscle cardiaque.

On va maintenant décrire un exemple de réalisation de la microsonde de l'invention, appliquée à la stimulation d'une cavité cardiaque par une sonde implantée dans le réseau coronaire.

Comme on l'a indiqué plus haut, cette application n'est aucunement limitative, et la microsonde de l'invention peut être utilisée dans un grand nombre d'autres applications compte tenu de son aptitude à être implantée dans des réseaux veineux, artériels ou lymphatiques profonds.

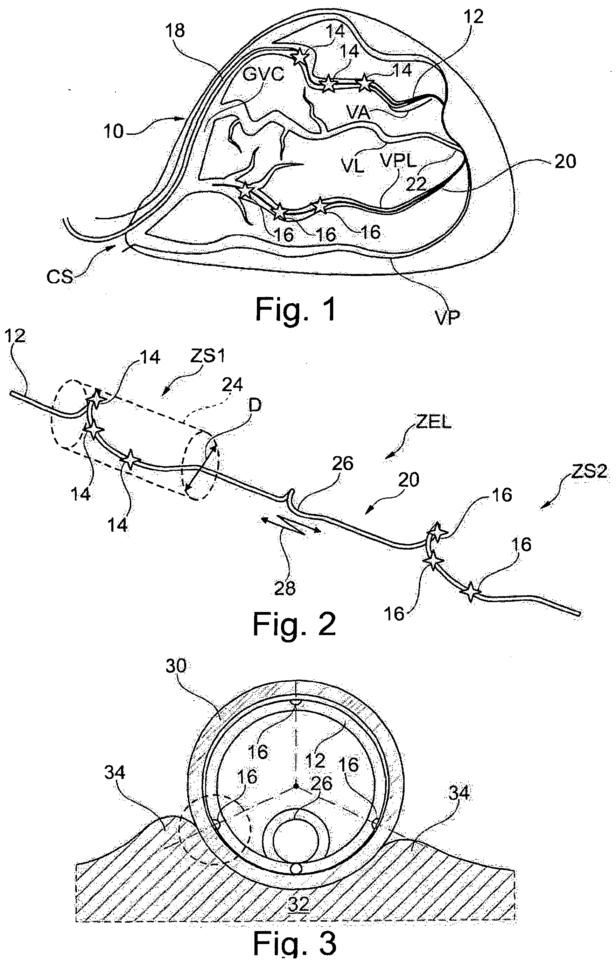

La Figure 1 illustre de façon générale le myocarde et les principaux vaisseaux du réseau coronaire, dans lequel on a introduit une sonde selon l'invention afin de stimuler le ventricule gauche.

Cette sonde est implantée par voie endocavitaire dans le réseau coronaire veineux via la veine cave supérieure, l'oreillette droite et l'entrée CS du sinus coronaire veineux. Le réseau coronaire veineux se développe ensuite en plusieurs branches à partir de la grande veine coronaire GVC, ces branches comprenant les veines postérolatérale VPL, latérale VL, antérolatérale VA et postérieure VP.

La référence 10 désigne de façon générale la sonde selon l'invention, qui comprend dans sa partie active un microcâble 12 dont la partie distale est représentée isolément Figure 2. La sonde 10 comprend en outre, dans sa région proximale, un microcathéter 18 pénétrant dans le sinus coronaire et la grande veine cardiaque GVC jusqu'au débouché de la veine antérolatérale VA.

Le microcâble 12 est introduit dans la veine antérolatérale VA et il porte une pluralité d'électrodes de stimulation 14 destinées à stimuler le ventricule gauche à partir de plusieurs sites situés dans cette veine VA. Le microcâble 12 porte également, à distance des électrodes 14, une autre série d'électrodes 16 destinées à stimuler le ventricule gauche depuis une autre veine, par exemple la veine postérolatérale VPL via une communication par une anastomose 22 reliant la veine antérolatérale VA et la veine postérolatérale VPL. Le microcâble traverse cette anastomose 22 et les régions les plus distales des deux veines VA et VPL le long d'une portion intermédiaire 20 dépourvue d'électrodes.

Grâce à cette configuration, il est possible non seulement de stimuler le ventricule gauche en plusieurs points de l'une des veines (du fait de la multiplication des électrodes 14 ou 16), mais en outre de prévoir deux zones relativement distantes de stimulation, respectivement la zone des électrodes 14 et la zone des électrodes 16, situées dans des régions proximales de deux veines différentes dans lesquelles il aurait été difficile de stabiliser ou fixer des sondes conventionnelles de stimulation du ventricule gauche, du fait du grand diamètre de l'embouchure de ces veines.

La Figure 2 décrit la partie distale, active du microcâble 12, avec la première série d'électrodes 14 et la seconde série d'électrodes 16, ces deux séries étant séparées par la partie intermédiaire 20.

La structure propre du microcâble 12 est par exemple celle décrite dans le EP 2 455 131 A1 précité auquel on pourra se référer pour de plus amples détails. Ce document donne également des indications sur le mode opératoire permettant d'implanter ce microcâble dans le réseau coronaire. Essentiellement, le microcâble 12 comprend un coeur électriquement conducteur pourvu d'un revêtement isolant sur toute sa longueur, à l'exception de zones ponctuellement dénudées servant à constituer les électrodes de détection/stimulation 14 et 16.

En ce qui concerne la structure du microcâble 12, le coeur de celui-ci est avantageusement une structure multifilaire dans laquelle chaque brin est de préférence constitué de nitinol (alliage NiTi) ou de MP35N-LT (35 % Ni, 35 % Co, 20 % Cr et 10 % Mo), matériaux dont l'avantage essentiel est leur extrême endurance en fatigue, avec enrobage par une gaine en platine iridié ou en tantale (pour la radioopacité et la biostabilité). Une telle structure permet d'optimiser la réponse aux exigences de résistance à la corrosion au niveau des électrodes et d'endurance à l'encontre des mouvements cardiaques. Ces microcâbles sont disponibles par exemple auprès de la société Fort Wayne Metals Inc., Fort Wayne, USA.

Le câble de coeur est revêtu d'une fine couche d'isolement, de l'ordre de 25 µm d'épaisseur, par exemple par co-extrusion sur le conducteur ou par échauffement d'un tube thermorétractable. L'isolant peut être :
- une fine couche de parylène (par exemple de type C) : dans ce cas, des fenêtres plus ou moins complexes sont aménagées le long du microcâble, par exemple par ablation plasma, pour former les électrodes 30 ; pour améliorer les performances électriques, ces zones peuvent être en outre revêtues par exemple de nitrure de titane ;
- un tube polyuréthanne interrompu aux endroits des électrodes 14 et 16;
- une ou plusieurs couches constituées de tubes en PET (polyéthylène téréphtalate), polymère fluoré, PMMA (polyméthacrylate de méthyle), PEEK (polyétheréthercétone), polyimide ou autre matériau similaire approprié.

Un avantage, particulièrement significatif, de cette structure résulte du caractère très souple et flottant *(floppy)* du microcâble, qui lui confère une excellente atraumaticité ; un tel microcâble agresse peu les tissus et préserve donc les cellules à proximité immédiate des électrodes.

Dans la configuration décrite ici, le microcâble comprend un conducteur unique, de sorte que les régions dénudées forment des électrodes qui, du point de vue électrique, sont reliées ensemble et se trouvent au même potentiel. Cette configuration monopolaire n'est cependant pas limitative, et l'invention est applicable aussi bien à une sonde multipolaire, avec un microcâble comprenant une pluralité de conducteurs distincts électriquement isolés entre eux, par exemple sous forme d'un faisceau de conducteurs isolés toronnés ensemble, chacun étant pourvu d'une ou plusieurs zones dénudées formant électrodes respectives.

Le microcâble présente une préforme tridimensionnelle à l'endroit des électrodes 14 qui forment une première zone de stimulation ZS1, ainsi qu'à l'endroit des électrodes 16 qui forment une seconde zone de stimulation ZS2. Cette préforme est destinée à favoriser le contact des électrodes avec la paroi du vaisseau, et par là même la performance électrique, comme cela est expliqué dans les EP 2 455 131 A1 et EP 2 581 107 A1 précités.

Les deux zones de stimulation ZS1 et ZS2 présentent une configuration identique, caractéristique de l'invention, que l'on va maintenant décrire.

La préforme tridimensionnelle du microcâble 12 à l'endroit de chacune de ces zones de stimulation est inscrite dans un volume enveloppe cylindrique 24 dont le diamètre D est choisi pour être, à l'état libre du microcâble, supérieur au diamètre du vaisseau cible, par exemple un diamètre de 12 mm pour une microsonde destinée à être implantée dans une veine cible de 2 à 6 mm de diamètre.

De préférence, la préforme tridimensionnelle est de forme hélicoïdale, inscrite sur cette enveloppe cylindrique 24. Ainsi, lorsque la sonde est introduite dans la veine, la forme hélicoïdale donnée du microcâble se plaquera naturellement sur les parois en assurant un contact permanent. Par ailleurs, les trois zones dénudées 14 ou 16 sont, en direction longitudinale, disposées en des emplacements respectifs régulièrement répartis sur le volume enveloppe cylindrique du microcâble 12.

Pour garantir la stabilité de l'ensemble, une préforme 26 est ajoutée dans la partie intermédiaire 20 dépourvue d'électrode, de manière à constituer une zone d'élongation ZEL permettant de faciliter l'élongation de la sonde dans la direction axiale (flèches 28) notamment lors des sollicitations exercées sur le microcâble à chaque battement cardiaque, ou si le patient bouge et exécute des mouvements amples (levée du bras, etc.). Ces efforts seront absorbés par une déformation de la zone d'élongation ZEL, sans déplacement des zones de stimulation ZS1 et ZS2, donc sans effet sur le positionnement des électrodes. La préforme 26 de la zone d'élongation ZEL est choisie de manière que son diamètre soit inférieur au diamètre de la veine cible, et sa conformation est choisie de manière à présenter une plus grande souplesse en direction axiale que celle des préformes des zones de stimulation ZS1 et ZS2. Cette zone d'élongation assure également une fonction de rétention complémentaire de la microsonde dans la veine.

La Figure 3 est une vue de bout, dans une direction axiale, de la microsonde dans la zone de stimulation ZS2, cette microsonde étant mise en place dans un vaisseau du réseau coronaire (représenté en coupe).

Sur cette vue, on peut voir que les électrodes 16 de la zone de stimulation ZS2 (il en est de même pour les électrodes 14 de la zone de stimulation ZS1) sont au nombre de trois et réparties à 120°, en vue axiale, sur la surface du volume enveloppe 24.

Cette configuration permet d'assurer en toute circonstance le contact d'au moins l'une des électrodes (l'électrode située en bas à gauche dans l'exemple de la Figure 3) avec la paroi intérieure du vaisseau 30, quelle que soit la configuration angulaire de la préforme portant les électrodes par rapport à la veine 30.

En effet, du point de vue anatomique, l'interface entre le muscle cardiaque 32 et la veine coronaire 30 ne se présente pas sous forme d'une surface plane, mais avec un repli latéral 34 (amas graisseux) faisant en sorte que l'étendue du contact entre la veine 30 et le muscle cardiaque 32 a lieu, anatomiquement, sur un secteur angulaire. Ceci augmente les chances de contact d'au moins une des électrodes 16 avec la paroi intérieure de la veine 30 dans la région de ce secteur angulaire, permettant ainsi une transmission directe de l'énergie électrique de l'électrode 16 au muscle cardiaque 32.

Le nombre et la répartition des électrodes 16 (trois électrodes, disposées à 120°) sont considérés comme préférentiels mais ne sont pas limitatifs : on pourrait prévoir un nombre plus élevé d'électrodes, par exemple au nombre de quatre, conduisant à une garantie supérieure d'un contact avec la paroi de la veine, mais au prix de pertes électriques plus importantes : dans ce cas, si une seule électrode vient en contact avec le tissu cardiaque, les trois quarts de l'énergie seront dissipés dans le sang et non vers le muscle cardiaque, contre les deux tiers seulement dans le cas d'une configuration à trois électrodes.

Par ailleurs, les zones dénudées constituant les électrodes 14 ou 16 ne s'étendent que sur un secteur angulaire du microcâble considéré en section droite, c'est-à-dire qu'il s'agit d'électrodes de type "sectorielles" tournées vers l'extérieur du volume-enveloppe 24 de la préforme, donc en direction de la paroi intérieure du vaisseau 30, dans la région où la possibilité de contact avec cette paroi sera maximale.

La surface dénudée de chacune des électrodes 14 ou 16 est typiquement constituée par un orifice formé dans le matériau de la gaine isolante sur un diamètre de 0,2 mm, par exemple par un tir laser de faible puissance permettant de fondre localement l'isolant du microcâble sans endommager le coeur conducteur de celui-ci.

La surface totale de l'ensemble des électrodes 14 de la zone de stimulation ZS1 (ou des électrodes 16 de la zone de stimulation ZS2) est alors inférieure à 0,1 mm², valeur 50 à 60 fois plus faible que celle des sondes conventionnelles telles que le modèle *Situs LV* décrit en introduction, dont la surface de l'électrode annulaire est d'environ 6 mm².

Il est ainsi possible de stimuler le muscle cardiaque en concentrant le champ électrique sur une zone réduite correspondant à cette faible surface dénudée, avec pour conséquence une augmentation importante de l'impédance de la sonde conduisant à une énergie délivrée beaucoup plus faible par le générateur à chaque impulsion de stimulation, pour une efficacité identique voire supérieure.

## Revendications

1. Une microsonde de détection/stimulation destinée à être implantée dans un vaisseau cible d'un réseau veineux, artériel ou lymphatique pour la stimulation d'un tissu dans la région du vaisseau cible, dans laquelle :
- la microsonde comporte une partie active distale constituée par un microcâble (12) de diamètre au plus égal à 0,66 mm (2 French) comprenant un coeur électriquement conducteur revêtu d'une couche d'isolement ;
- le microcâble comprend au moins une zone de stimulation (ZS1, ZS2) où la couche d'isolement présente une pluralité de zones dénudées (14, 16) formant des électrodes de stimulation respectives ;
- le microcâble présente dans la zone de stimulation une préforme tridimensionnelle configurée de manière que le microcâble épouse la paroi du vaisseau cible (30) dans la zone de stimulation ; et
- la préforme tridimensionnelle est inscrite dans un volume enveloppe cylindrique (24) dont le diamètre (D) est choisi pour être, à l'état libre du microcâble, supérieur au diamètre du vaisseau cible,
**caractérisée en ce que** :
- le microcâble comporte dans la région de la préforme tridimensionnelle au moins trois desdites zones dénudées, ces au moins trois zones dénudées étant disposées en des emplacements respectifs du microcâble situés sur ledit volume enveloppe cylindrique, ces emplacements étant en outre régulièrement répartis sur la circonférence du volume enveloppe cylindrique considéré en projection axiale ; et
- la surface dépourvue de revêtement isolant de chaque zone dénudée (14, 16) est inférieure à 0,3 mm².

2. La microsonde de la revendication 1, dans laquelle la surface dépourvue de revêtement isolant de chaque zone dénudée (14, 16) est inférieure à 0,1 mm².

3. La microsonde de la revendication 1, dans laquelle le microcâble comporte trois zones dénudées (14, 16) disposées en des emplacements respectifs répartis à 120° sur la circonférence du volume enveloppe cylindrique considéré en projection axiale.

4. La microsonde de la revendication 1, dans laquelle :
- les zones dénudées (14, 16) ne s'étendent que sur un secteur angulaire du microcâble considéré en section droite ; et
- le secteur angulaire est tourné vers l'extérieur du volume enveloppe de la préforme.

5. La microsonde de la revendication 1, dans laquelle la préforme tridimensionnelle (24) est une préforme hélicoïdale.

6. La microsonde de la revendication 1, dans laquelle les au moins trois zones dénudées (14, 16) sont disposées en des emplacements respectifs du microcâble régulièrement répartis en direction longitudinale sur le volume enveloppe cylindrique.

7. La microsonde de la revendication 1, comprenant deux zones de stimulation distinctes (ZS1, ZS2) séparées par une zone intercalaire (ZEL) d'élongation et de rétention, conformée de manière à rendre la microsonde élastiquement déformable dans le sens longitudinal (28) sous l'effet des sollicitations de traction/compression axiale exercée sur la microsonde dans sa région proximale par rapport à la zone d'élongation.

## Patentansprüche

1. Detektions-/Stimulationsmikrosonde, die zur Implantation in ein Zielgefäß eines Venen-, Arterien- oder Lymphsystems bestimmt ist, um ein Gewebe in der Region des Zielgefäßes zu stimulieren, wobei:
- die Mikrosonde einen distalen aktiven Teil aufweist, der aus einem Mikrokabel (12) mit einem Durchmesser von höchstens 0,66 mm (2 French) besteht, das einen elektrisch leitenden Kern aufweist, der mit einer Isolierungsschicht ummantelt ist;
- das Mikrokabel mindestens einen Stimulationsbereich (ZS1, ZS2) aufweist, in dem die Isolierungsschicht eine Vielzahl von abisolierten Bereichen (14, 16) aufweist, die jeweils Stimulationselektroden bilden;
- das Mikrokabel in dem Stimulationsbereich eine dreidimensionale Vorform aufweist, die derart konfiguriert ist, dass sich das Mikrokabel an die Wand des Zielgefäßes (30) in dem Stimulationsbereich anpasst; und
- die dreidimensionale Vorform in einem zylinderförmigen Hüllvolumen (24) enthalten ist, dessen Durchmesser (D) derart ausgewählt ist, dass er, wenn sich das Mikrokabel in freiem Zustand befindet, größer ist als der Durchmesser des Zielgefäßes,
**dadurch gekennzeichnet, dass**:
- das Mikrokabel in der Region der dreidimensionalen Vorform mindestens drei der abisolierten Bereiche aufweist, wobei diese mindestens drei abisolierten Bereiche an Stellen des Mikrokabels angeordnet sind, die jeweils auf dem zylinderförmigen Hüllvolumen liegen, wobei diese Stellen, bei Betrachtung in axialer Projektion, ferner regelmäßig auf dem Umfang des zylinderförmigen Hüllvolumens verteilt sind; und
- die Oberfläche ohne isolierende Ummantelung jedes abisolierten Bereichs (14, 16) kleiner als 0,3 mm² ist.

2. Mikrosonde nach Anspruch 1, wobei die Oberfläche ohne isolierende Ummantelung jedes abisolierten Bereichs (14, 16) kleiner als 0,1 mm² ist.

3. Mikrosonde nach Anspruch 1, wobei das Mikrokabel drei abisolierte Bereiche (14, 16) aufweist, die an Stellen angeordnet sind, die, bei Betrachtung in axialer Projektion, jeweils mit 120° auf dem Umfang des zylinderförmigen Hüllvolumens verteilt sind.

4. Mikrosonde nach Anspruch 1, wobei:
- sich die abisolierten Bereiche (14, 16) nur über einen Winkelabschnitt des betrachteten Mikrokabels im Querschnitt erstrecken; und
- der Winkelabschnitt auf die Außenseite des Hüllvolumens der Vorform gedreht ist.

5. Mikrosonde nach Anspruch 1, wobei die dreidimensionale Vorform (24) eine schraubenförmige Vorform ist.

6. Mikrosonde nach Anspruch 1, wobei die mindestens drei abisolierten Bereiche (14, 16) an Stellen des Mikrokabels angeordnet sind, die jeweils regelmäßig in Längsrichtung auf dem zylinderförmigen Hüllvolumen verteilt sind.

7. Mikrosonde nach Anspruch 1, die zwei verschiedene Stimulationsbereiche (ZS1, ZS2) aufweist, die durch einen Zwischenbereich (ZEL) zur Verlängerung und zum Halten getrennt sind, der derart angepasst ist, dass er die Mikrosonde in Längsrichtung (28) unter der Einwirkung von Zugbelastungen/axialem Druck, der auf die Mikrosonde in ihrer in Bezug auf den Verlängerungsbereich proximalen Region ausgeübt wird, elastisch verformbar macht.

## Claims

1. A detection/stimulation micro-lead intended to be implanted in a target vessel of a venous, arterial or lymphatic system for the stimulation of a tissue in the region of the target vessel, wherein:
- the micro-lead includes a distal active part consisted by a micro-cable (12) of diameter at most equal to 0.66 mm (2 French), comprising an electrically conductive core coated with an insulation layer;
- the micro-cable comprises at least one stimulation zone (ZS1, ZS2) where the insulation layer has a plurality of naked zones (14, 16) forming respective stimulation electrodes;
- the micro-cable has, in the stimulation zone, a three-dimensional preform configured so that the micro-cable conforms the wall of the target vessel (30) in the stimulation zone; and
- the three-dimensional preform is inscribed in a cylindrical envelope volume (24) whose diameter (D) is chosen so that, in the free state of the micro-cable, greater than the diameter of the target vessel, **characterized in that**:
- the micro-cable includes, in the region of the three-dimensional preform, at least three of said naked zones, these at least three naked zones being arranged at respective places of the micro-cable located on said cylindrical envelope volume, these places being further regularly distributed over the circumference of the cylindrical envelope volume considered in axial projection; and
- the surface devoid of insulating coating of each naked zone (14, 16) is lower than 0.3 mm².

2. The micro-lead of claim 1, wherein the surface devoid of insulating coating of each naked zone (14, 16) is lower than 0.1 mm².

3. The micro-lead of claim 1, wherein the micro-cable includes three naked zones (14, 16) arranged at respective places distributed at 120° over the circumference of the cylindrical envelope volume considered in axial projection.

4. The micro-lead of claim 1, wherein :
- the naked zones (14, 16) extend only over an angular sector of the micro-cable considered in cross section; and
- the angular sector is turned towards the outside of the envelope volume of the preform.

5. The micro-lead of claim 1, wherein the three-dimensional preform (24) is a helical preform.

6. The micro-lead of claim 1, wherein the at least three naked zones (14, 16) are arranged at respective places of the micro-cable regularly distributed in the longitudinal direction over the cylindrical envelope volume.

7. The micro-lead of claim 1, comprising two distinct stimulation zones (ZS1, ZS2) separated by an intermediate zone (ZEL) of elongation and retention, shaped so as to make the micro-lead elastically deformable in the longitudinal direction (28) under the effect of the axial traction/compression stresses exerted on the micro-lead in its proximal region with respect to the elongation zone.
